# EUROPEAN PATENT APPLICATION

(11) **EP 0 578 138 A2**
(43) Date of publication of application: **12.01.1994**
(21) Application number: 93110550.6
(22) Date of filing: 01.07.1993
(51) Int. Cl.: C12Q 1/68, C12N 9/12

(54) **Gene-detecting method**

(30) Priority: 10.07.1992 JP 183452/92
(71) Applicant: HITACHI, LTD., Chiyoda-ku, Tokyo 100 (JP)
(72) Inventor: Kambara, Hideki, Hachioji-shi (JP); Okano, Kazunori, Shiki-shi (JP); Murakawa, Katsuji, Kokubunji-shi (JP)
(74) Representative: Strehl Schübel-Hopf Groening & Partner

(57) **Abstract**

A fluorescence-labelled DNA probe 11 is hybridized with a specimen 7 to form 9 double-stranded hybrid 8; successive decomposition and separation of the hybridized DNA probe with the aid of an enzyme specifically decomposing the double-stranded hybrid 8 are repeated to high speed-decompose the DNA probe alone hybridized to the specimen DNA; and after a shortened DNA probe is amplification-produced in this way, the shortened DNA probe 20 and unreacted DNA probe 21 are separately discriminated and detected to detect the specimen DNA 7 with high sensitivity. The shortened DNA probe can be produced in amounts which are larger by several figures than the amount of the specimen DNA in several minutes and without raising and lowering the reaction temperature, so that the specimen DNA can be detected rapidly.

## Description

### BACKGROUND OF THE INVENTION

### Field of the Invention

The present invention relates to a high-sensitivity detecting method and detecting apparatus for genes or DNA.

### Description of the Related Art

A method of diagnosing diseases by discriminating particular base sequences on genes, DNA diagnosis or gene diagnosis is now widespread. Usually, the number of copies of DNA which is a target of detection is so small that a high-sensitivity detecting method is required. Hitherto, for detection of trace amounts of DNA, DNA probes labelled with a radioactive isotope have been used. That is, DNA, a specimen, is subjected to electrophoresis on an agarose gel, separated by the size and the separated DNA pattern is transferred and fixed to a cellulose filter. Then, a radioactive isotope-labelled DNA probe is sprinkled over the pattern to hybridize with DNA having a complementary sequence. On closely adhering the filter to a film, etc., {3-ray, etc. radiate from the hybridization site of the DNA probe, as a result of which the site is transferred to the film to enable the detection of DNA. This detection using radioactive isotope labelling is highly sensitive, but it has a problem that handling is troublesome. Because of this, a technique using an enzymatic reaction for amplifying the number of copies of a DNA section having a sequence intended to detect, i.e. a polymerase chain reaction (PCR method), is developed and used [literature: Science 242, 229 (1988)]. This method includes hybridizing complementary strand-synthesizing primers with both sides of double-stranded DNA having the desired region therebetween, and repeating complementary strand synthesis with DNA polymerase. This method enables 10⁵ to 10⁶⁻fold amplification, so that sufficient detection of DNA becomes possible even with a fluorescent label, etc. In the PCR method, the complementary strand synthesis, separation of the double-stranded DNA and hybridization of the primers are repeated with a heat-resistant enzyme and a heat-cycling apparatus for repeating rise and lowering in temperature, so that two primers amplifying the DNA complementary strand are indispensable.

### SUMMARY OF THE INVENTION

In the PCR amplification method, DNA copies are first produced with the desired DNA as a template, but after several cycles, the synthesized copies become the template. Therefore, the number of copies increases by geometrical progression. Because of this, if there is a site, if a few, for the amplifying primer to hybridize therewith, the amplification of DNA begins and thereafter proceeds automatically, so that this method has a weak point that it is easily affected by contamination, etc. Also, this method has problems that for carrying out the amplification, there is a necessity to prepare for two primers, and further that in detecting virus having a large variation, there is difficulty in searching an advantageous and convenient amplification region for detecting virus. For this reason, there has been a demand for developing a high-sensitivity method for detecting desired DNA sections in place of the PCR amplification method. In view of the situation described above, an object of the present invention is to provide a method for amplifying a DNA probe carrying the information of specimen in place of the PCR amplification method, and realize a high-sensitivity gene-detecting method and gene-detecting apparatus.

The above object can be attained by using an enzyme decomposing the double-stranded portion of DNA or RNA by snipping the portion from its end, making fluorophor labelled oligomer probes hybridized on specimen DNA, shortening the DNA or RNA probe in the presence of a specimen DNA or RNA, and detecting the shortened DNA or RNA probe. As the enzyme decomposing the above double-stranded portion from its end, there can be used enzymes having an exonuclease activity such as exonuclease III, \ exonuclease, exonuclease Bal 31 and the like. In short the present invention is characterized by using an enzyme having an exonuclease activity decomposing the double-stranded DNA or RNA or DNA-RNA hybrid from its end which contains a DNA probe comprising a portion having a weak hybridization power labelled with a fluorescent body or dye and a portion of strong hybridization power having a sequence complementary to the specimen.

More specifically, the gene-detecting method of the present invention comprises the step of hybridizing a DNA or RNA probe with a specimen DNA or RNA, respectively, the step of successively decomposing the nucleotides of the double strand from its end by the use of an enzyme having an exonuclease activity and the step of detecting the shortened DNA or RNA probe. In this method, a DNA or RNA probe labelled with a fluorescence or dye can be used.

Also, the gene-detecting method of the present invention comprises hybridizing a plural number of DNA or RNA probes which are mutually different in length from the end to the position of the fluorescence-labelled base of the DNA or RNA probes with a specimen DNA or RNA, respectively, successively decomposing the nucleotides of the double strand from its end by the use of an enzyme having an exonuclease activity, thereby changing the molecular weights of the shortened DNA or RNA probes so that the electrophoretic mobility changes for each DNA or RNA probe, and discriminating the DNA or RNA probes shortened by the action of the enzyme having an exonuclease activity from the specimen DNA or RNA, thereby separately detecting the plural sites of the specimen DNA or RNA having different base sequences. The present invention is characterized in that, in every DNA or RNA probe, the decomposition by the enzyme having an exonuclease activity is made not to proceed over the base at the predetermined position on the above DNA or RNA probes.

Further, the gene-detecting method of the present invention comprises hybridizing a plural number of DNA or RNA probes which are mutually different in length from the end to the position of the fluorescence-labelled base of the DNA or RNA, with plural kinds of specimen DNA or RNA, respectively, successively decomposing the nucleotides of the double strand from its end by the use of an enzyme having an exonuclease activity, thereby changing the molecular weights of the shortened DNA or RNA probes so that the migration rate changes for each DNA or RNA probe, and discriminating the DNA or RNA probes shortened by the action of the enzyme having an exonuclease activity from plural kinds of specimen DNA or RNA, thereby separately detecting the plural kinds of specimen DNA or RNA. The present invention is characterized in that, in every DNA or RNA probe, the base at the predetermined position on the DNA or RNA probe is chemically modified or the degree of the stabilization of the hybridization is controlled so that decomposition by the enzyme having an exonuclease activity does not proceed over the base at that position.

Further, the gene-detecting method of the present invention comprises hybridizing a plural number of DNA or RNA probes which are mutually different in kind of fluorescent label of the DNA or RNA probe, with plural kinds of specimen DNA or RNA, respectively, successively decomposing the nucleotides of the double strand from its end by the use of an enzyme havaing an exonuclease activity, and discriminating the shortened DNA or RNA probes, and detecting the plural kinds of specimen DNA or RNA, or comprises hybridizing DNA or RNA probes having different base sequences and labelled with a plural number of different fluorescent labels with plural sites of a specimen DNA or RNA having different base sequence portions, respectively, shortening the hybrids with an enzyme having an exonuclease activity, and separately detecting the plural sites of the specimen DNA or RNA having different base sequence portions.

In the gene-detecting methods explained above: The length of the DNA or RNA probe shall be not less than 8 and not more than 100 bases.

The gene-detecting apparatus of the present invention is one utilizing the gene-detecting method explained above, having a means to discriminate the shortened DNA or RNA probe, and detecting one or plural kinds of specimen DNA or RNA.

By using the above methods and apparatus of the present invention, DNA diagnosis and DNA- detecting apparatus become possible.

The fluorescence-labelled DNA probe hybridizes with the specimen DNA to form a hybrid having a double strand. When an enzyme having an activity to snip a base at the end of the double-stranded DNA, i.e. an enzyme having an exonuclease activity (e.g. exonuclease III, \ exonuclease, exonuclease Bal 31, etc.) is allowed to act on this hybrid, the nucleotides are successively snipped out of the 3'-end or 5'-end of the double-stranded portion. In order that the DNA probe may hybridize and stably exist in the form of a double strand, it is necessary for the DNA probe to have a definite length. However, when the length becomes short by the successive snipping-off of the nucleotides from the end of the strand, the shortened DNA probe (in some cases, only the fluorescence-labelled portion remains) leaves the specimen DNA. The fresh DNA probe molecule present in excess hybridizes in turn with the residual specimen DNA to form a double strand, which is again decomposed by the enzyme having an exonuclease activity, i.e. an exonuclease. Thus, the length of the DNA probe becomes short in the presence of the specimen DNA and an exonuclease. Thus reaction proceeds irreversibly, so that after a definite period of time, shortened fluorescence-labelled DNA probes which are far more than the number of copies of the specimen DNA are formed. By subjecting the shortened fluorescence-labelled DNA probe to electrophosis to separate it by length and detecting the separated fractions, the presence or absence of the specimen DNA can be examined with high sensitivity. In order to raise the accuracy of the examination, it is also possible to hybridize fluorescence-labelled DNA probes having different base sequences with plural sites of the specimen DNA, shorten the resulting hybrids and separately detect the plural sites of the specimen DNA having different base sequences. In other words, by changing the kind of a fluorescent body for labelling these probes or the length of the portion of weak hybridization power labelled with a fluorescent body or dye so that the size of the DNA probes after shortening may change, it is possible to separate them by the path length of electrophoresis, etc. thereby separately detecting the separated probes.

In short, the desired shortened DNA probe can be produced as being amplified by hybridizing the fluorescence-labelled DNA probe with the specimen to form a double strand, and repeating the decomposition and separation of the DNA probe by the use of an enzyme specifically decomposing the double strand, thereby high speed-decomposing the DNA probe alone hybridized to the specimen DNA.

According to the present invention, as described above, by complementarily bonding the specimen DNA with the DNA probe having a sequence complementary to the specimen DNA, the complementarily bonded DNA probe can selectively be shortened. This reaction is catalyzed by the enzyme, and the shortened DNA probe can be produced in amounts far larger than the number of copies of the specimen DNA. By separating the resulting shortened DNA probe from the unreacted one, the specimen DNA can be detected with high sensitivity. And thus, high-sensitivity detection of gene becomes possible. The reaction of the present invention can produce the shortened DNA probe in amounts which are larger, for example, by 2 to 3 figures than the amount of the specimen DNA in several minutes and without raising and lowering the temperature. Consequently, the method and apparatus of the present invention are effective for rapid detection of a specimen DNA.

### BRIEF DESCRIPTION OF THE DRAWINGS

Figs. 1A to 1F are views illustrating examples of the structure of various types of fluorescence-labelled DNA probe used in the examples of the present invention.
Figs. 2A to 2F are typical views illustrating the process of the present invention in which the number of DNA probes shortened by the enzymatic reaction is amplified.
Fig. 3 is a view illustrating one example of the electrophoretic pattern of the product obtained by the amplification reaction of the present invention.
Figs. 4A and 4B show examples of hybrids in a case where the amplification of the number of shortened DNA probes is carried out using plural number of DNA probes.

### PREFERRED EMBODIMENTS OF THE INVENTION

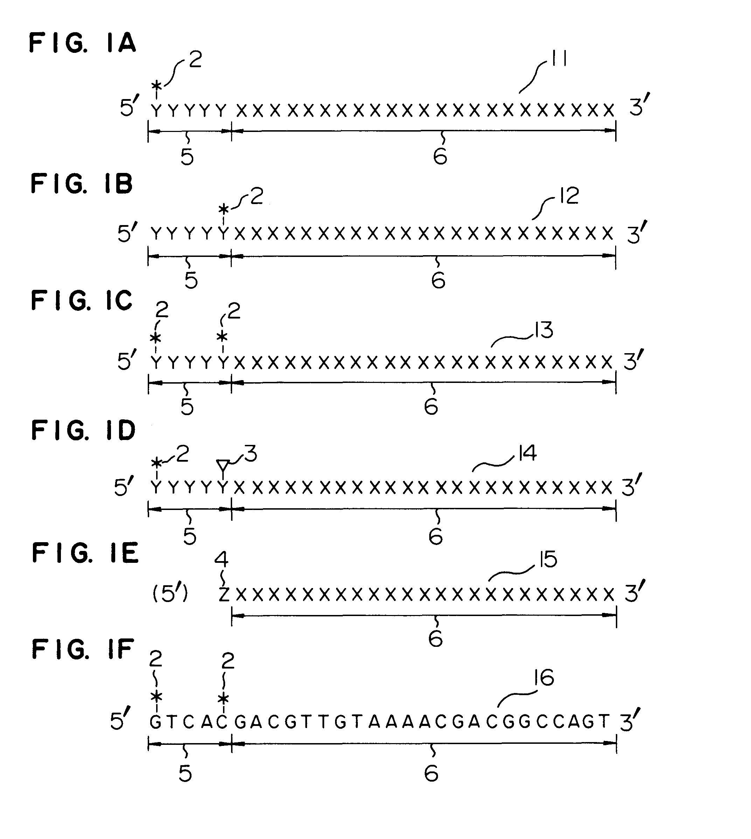

The present invention will be illustrated with reference to the drawings. As an example of the specimen was used phage M13 which is an easily available single-stranded DNA. Here, a single-stranded DNA will be shown as example, but detection can be likewise carried out if the specimen is a single-stranded RNA such as virus, etc. First, a DNA probe having a sequence complementary to the specimen is produced. In the case of virus showing a large variation, the DNA probe is produced by selecting a region having a well-stored base sequence. In the present examples, various types of fluorescence-labelled DNA probe as shown in Figs. 1A to 1E can be used. The fluorescence-labelled DNA probes used in the present examples, as shown in Fig. 1A to Fig. 1E, comprise a hybridization region 6 which is a portion well hybridizing with the specimen DNA and a labelling region 5 of weak hybridization power labelled with a dye or fluorescent label 2. In Fig. 1A to Fig. 1 E, X and Y each represents a base A, C, G or T, and Z represents the molecular chain of an organic substance bonded with a dye or fluorescent label. The DNA probe 11 in Fig. 1A has a fluorescent label 2 at the 5'-end; the DNA probe 12 in Fig. 1B has a fluorescent label 2 at the fifth base from the 5'-end; the DNA probe 13 in Fig. 1C has a fluorescent label 2 at both the 5'-end and the fifth base from the 5'-end; and the DNA probe 14 in Fig. 1D has a fluorescent from the fluorescent label 2, at the fifth base from the 5'-end. In Fig. 1A, Fig. 1 B and Fig. 1 D, the labelling region 5 may further be labelled with one or plural fluorescent labels 2 or 3. Further, in Fig. 1A to Fig. 1D, the labelling region 5 may contain inosine which has a weak hybridization power. The DNA probe 15 in Fig. 1 E is an example in which the molecular chain Z of an organic substance bonded with a fluorescent label has been bonded to the 5'-end of the hybridization region 6. The fluorescent label bonded to the molecular chain Z of an organic substance may be one in which one or plural fluorescent labels of the same kind have been bonded together, or one or plural fluorescent labels of plural kinds have been bonded together.

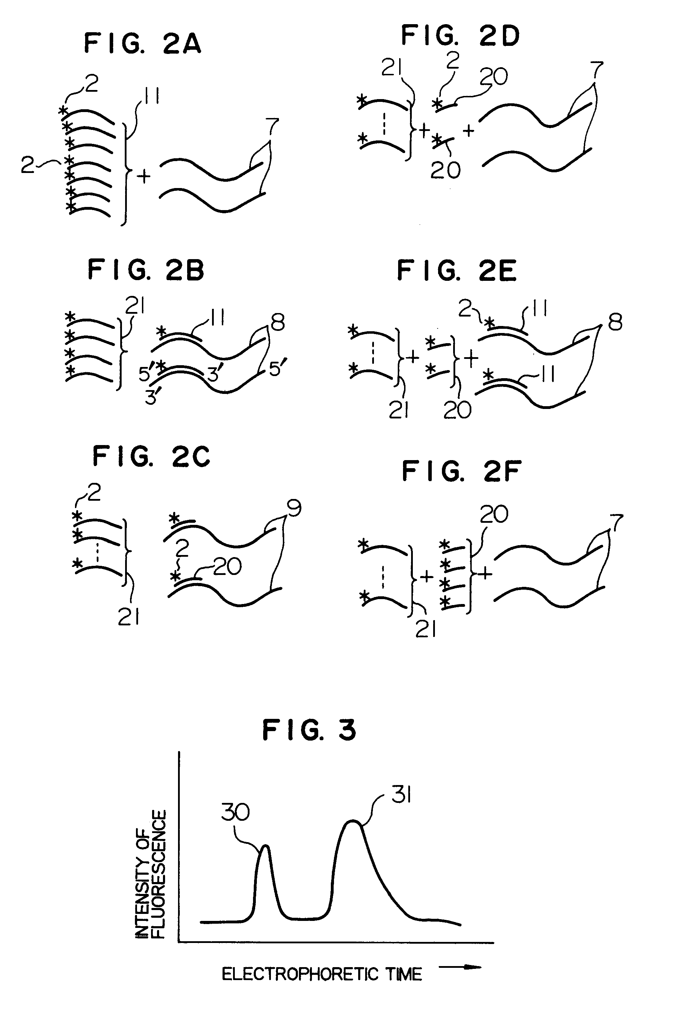

The first example of the present invention will be illustrated with reference to a case where the DNA probe 11 in Fig. 1A is used. In Fig. 2A to Fig. 2F, a process in which the shortened DNA probe is amplification-produced by hybridizing the fluoresecnce-labelled DNA probe with the specimen to form a double strand, and repeating the decomposition and separation of the DNA probe by the use of an enzyme specifically decomposing the double strand, thereby high speed-decomposing the DNA probe alone hybridized with the specimen DNA, is shown by a typical view. A reaction occurring in such a process will be illustrated with Fig. 2A to Fig. 2F. As shown in Fig. 2A, the single-stranded specimen 7 and the DNA probe 11 (only one fluorescent label 2 has been bonded to the 5'- end) in Fig. 1A are mixed, and on holding the resulting mixture under predetermined conditions, the hybridization reaction begins. As a result, as shown in Fig. 2B, the hybrid 8 of the DNA probe 11 with specimen 7 and the unreacted DNA probe 21 are produced by the reaction. Since the number of the specimens 7 is small and a sufficient excess of the DNA probe 11 over the number of the specimens 7 is used, the unreacted DNA probe 21 remains in excess. Under this condition, exonuclease III acting to ship the nucleotides from the 3'-end of DNA is added. By the catalytic action of exonuclease III, the nucleotides of the DNA probe 11 constituting the double-stranded portion of the hybrid 8 are successively snipped from the 3'-end of the DNA (the end-decomposition reaction does not proceed at the 3'-end of the specimen DNA constituting the double-stranded portion of the hybrid 8 because of the presence of the fluorescent label 2). As shown in Fig. 2C, the length of the DNA probe 11 is shortened to turn shortened DNA probe 20. When the hybrid 9 of the shortened DNA probe 20 with specimen 7 becomes weak in hybridization power and comes to fail to stably exist under the given conditions, the shortened DNA probe 20 labelled with the fluorescent label 2 separates from the hybrid 9 to become free. The shortened DNA probe 20, when the number of its hybridizing base pairs becomes small, becomes weak in power to hybridize with the specimen DNA (there is also a case where the shortened DNA probe 20 hybridizes with the single-stranded specimen 7 at its 5'-end alone lavelled with the fluorescent label 2). As a result, as shown in Fig. 2D, the hybrid 9 breaks to turn mixed state comprising the single-stranded specimen 7, an excess of the unreacted DNA probe 21 and the free shortened DNA probe 20 (there is also a case where the probe 20 comprises the 5'-end alone labelled with the fluorescent label 2). This mixed state is substantially the same as the state of Fig. 2A except that the free shortened DNA probe 20 is present. Therefore, as shown in Fig. 2E, the single-stranded specimen 7 again forms the hybrid 8, and the nucleotides of the DNA probe 11 constituting the double-stranded portion of the hybrid 8 are successively snipped from the 3'-end by the catalytic action of exonuclease III. In the same manner as in Fig. 2C, the length of the DNA probe 11 is shortened to form a shortened DNA probe 20 which separates from the specimen 7 to become free. Thus, the number of the shortened DNA probes 20 amplifies as shown in Fig. 2F. This amplification continues while an excess of the unreacted DNA probe 21 is present and exonuclease III keeps the enzymatic activity.

The second example of the present invention will be illustrated with reference to a case where the DNA probe 16 in Fig. 1 F is used. In this example, the DNA probe 16 having a structure shown in Fig. 1 F, i.e. a 28-mer DNA probe was used. The fluorescent label 2 was put in the 5'-end and fifth base from the 5'-end. The fluorescent label may be put in the 5'-end alone or bases other than the fifth base. The fluorescent label molecule 2 has been attached to the 5'-end and the fifth base from the 5'-end, so that the power of this portion to hybridize with the specimen DNA is weak. This portion is called a labelling region. A plural number of DNA probes can be discriminated from one another by changing the length of the portion of this labelling region. Also, this labelling region 5 can be made easy to separate from the specimen DNA after end-decomposition by an exonuclease, by introducing inosine having a weak hybridization power into this region, or forming this region with the molecular chain of an organic substance which makes a difference in electrophoretic mobility due to its length, but has no hybridization power, and to which a fluorescent label has been bonded. The DNA probe has a complementary DNA comprising 23 mers at the side of the 3'-end, and this region is a portion well hybridizing with the specimen DNA. This region is referred to as hybridization region hereinafter. The total length of this hybridization region and fluorescent labelling region, i.e. the length of the DNA probe, is preferably not less than 8 to 10 mers and not more than 100 mers in order to stably hybridize the DNA probe with the specimen DNA at room temperature. The reason for this is that a further increase in the total length raises a probability to cause the self-hybridization reaction of the DNA probe itself. When the hybridization is carried out at a higher temperature, the length needs to be made large. In this example, the length was made 23 mers in order to form a sufficiently stable double strand. The selection of the working temperature is important, a preferred temperature being one at which the hybridization occurs if the hybridization region of the DNA probe completely fits the complementary strand of the specimen DNA, but at which the bonding does not occur at all when the above region does not fit the above strand or the DNA probe has turned short probe comprising the labelling region alone. From this standpoint, the working temperature is preferably a little higher and hence a heat-resistant exonuclease is preferably used.

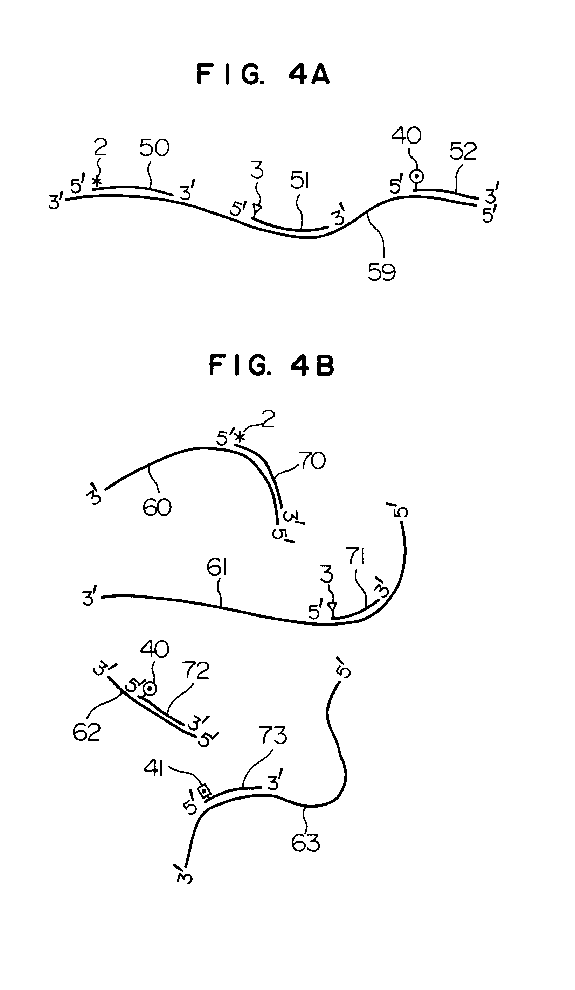

A TBE buffer solution containing several hundred copies of the specimen and 0.5 l1.,l of a solution containing 0.1 pmole of the DNA probe were mixed, and hybridization reaction was carried out under predetermined conditions. Thereafter, 1 unit of exonuclease III was injected into the solution, and reaction was carried out for 10 minutes. One unit of exonuclease III has an ability to act on the 3'-end of the double-stranded DNA, thereby dissociating about 1 nmole of a nucleic acid molecule in 30 minutes. The amplification of the number of shortened DNA probes follows completely the same principle as explained in the first example. The reaction product is separated from the unreacted DNA probe by separation means such as liquid chromatography, gel electrophoresis, capillary electrophoresis, etc., and then detected with a fluorescence detector. When the gal electrophoresis is used, a real time fluorescence-type DNA sequencer and the like can also be used, but 4 to 6 cm of a migration path length will suffice. In this example, electrophoresis on 6% acrylamide gel was used. That is, a gel plate of short migration path lengh was attached to a DNA sequencer, and 1 l1.,l of the reaction product was injected into an electrophoretic well. The migration path length of the separated probe was determined as 6 cm. Fig. 3 shows the result of measurement. The electrophoretic peak 30 due to the shortened DNA probe 20 and that 31 due to the unreacted DNA probe 21 were detected. Since, however, the electrophoretic mobility of the shortened DNA probe 20 was different from that of the unreacted DNA probe 21, the measurement of the shortened DNA probe was not hindered by the unreacted DNA probe present in excess. Further, the separation becomes easy when the original length of the DNA probe is made larger. As described above, by keeping almost the same degree of stability as in the hybridization and controlling the length of the labelling region through the length of a linker portion spanning from the region to inosine or a fluorescent molecule, or by changing the length of the labelling region or kind of the label (e.g. kind of fluorescent body) so that the molecular weight of the shortened DNA probe changes and as a result, the migration rate changes for each DNA probe, or that the kind of a signal from the lable (e.g. fluorescent wave length) changes for each DNA probe, it is possible to discriminate what the DNA probe shortened by the specimen and enzyme is, thereby detecting the plural kinds of specimen at the same time. Of course, one kind of specimen can be examined on plural test items using the same probe. Fig. 4A shows hybrids which are in the conditons of Fig. 2B or Fig. 2E in a case where DNA probes 50, 51 and 52 labelled with different fluorescent labels 2, 3 and 40 are hybridized with the plural portions, respectively, of a specimen 59 and examined for plural test items at the same time. In Fig. 4A, as explained above, by changing the length of the labelling region of every probe so that the molecular weight of every shortened probe changes and as a result, the migration rate changes for every DNA probe, what the DNA probes shortened by the specimen and enzyme are may be discriminated. Fig. 4B shows hybrids which are in the conditions of Fig. 2B or Fig. 2E in a case where plural, different specimens 60, 61, 62 and 63 are tested at the same time. The specimens form hybrids with DNA probes 70, 71, 72 and 73 labelled with different fluorescent markers 2, 3, 40 and 41, respectively. The hybrids shown in Fig. 4A or Fig. 4B are treated by the catalytic action of exonuclease III as follows: As described in Fig. 2A to Fig. 2F, the nucleotides of the DNA probe of every hybrid are successively snipped from the 3'- end to produce a shortened DNA probe which separates from the hybrid to become free, and thus the number of the shortened DNA probes are amplified.

The rate of formation of the shortened DNA probe by exonuclease is proportional to the number of copies of the specimen DNA, so that it is easy to carry out quantitative evaluation. For example, when the amount of the specimen DNA is 0.1 pmole, the shortened DNA probe can be produced in amounts which are larger by 2 to 3 figures than the amount of the specimen DNA in several minutes.

Since exonuclease III doesnot act on single-stranded DNA or RNA, only the hybridized DNA probe becomes a target of decomposition.

The decomposition and separation can be promoted by using a heat-resistant enzyme, raising the working temperature and using a longer probe, and if necessary raising and lowering the temperature. In the description of the above examples, the enzyme used to catalyze the process in which the nucleotides are successively snipped from the end of the DNA molecule, was exonuclease III which snips the nucleotides from the 3'-end of DNA. However, the same as above is also possible using \ exonuclease which snips the nucleotides from the 5'-end of DNA, or exonuclease Bal 31 which decomposes the nucleotides from both the 3'- and 5'- ends of DNA. The above fluorescent marker which can be used includes fluorescein isothiocyanate (FITC), sulforhodamine 101, rhodamine derivatives, tetramethylrhodamine isothiocyanate (TRITC) and the like.

## Claims

1. A gene detecting method comprising the step of using an enzyme which decomposes a double stranded portion of DNA or RNA from the end of the portion.

2. A gene detecting method according to claim 1, wherein at least one strand of the double stranded portion of DNA or RNA is a DNA or RNA oligomer labelled with a marker.

3. A gene detecting method according to any of the claims 1 or 2, wherein said enzyme has exonuclease activity.

4. A gene detecting method comprising the steps of shortening a DNA or RNA probe from its end in the presence of a specimen DNA or RNA, respectively, and detecting the shortened DNA or RNA probe.

5. A gene detecting method according to claim 4, wherein the DNA or RNA probe is labelled with a marker.

6. A gene detecting method according to any of the claims 4 or 5, wherein said DNA or RNA probe is shortened by the use of an enzyme having exonuclease activity.

7. A gene detecting method according to any of the claims 4 to 6, wherein the DNA or RNA probe is hybridized with a specimen DNA or RNA prior to shortening and detecting the DNA or RNA probe.

8. A gene detecting method according to any of the claims 4 to 7, wherein the shortened and labelled DNA or RNA probe is separately detected by electrophoresis.

9. A gene detecting method comprising the steps of
hybridizing a plurality of DNA or RNA- probes, which are mutually different in length from the end position of the labelled base of the DNA or RNA probes, with a specimen DNA or RNA, respectively, to obtain hybrids,
successively decomposing the nucleotides of the double strand from its end by the use of an enzyme having exonuclease activity,
discriminating the labelled DNA or RNA probes shortened by the action of the enzyme from the specimen DNA or RNA in terms of electrophoretic mobility, and
separately detecting plural sites of the specimen DNA or RNA which sites have different base sequences.

10. A gene detecting method according to claim 9, wherein the DNA or RNA probes, which are mutually different in length from the end to the position of the labelled base, are hybridized with plural kinds of specimen DNA or RNA, thereby detecting the plural number of specimen DNA or RNA.

11. A gene detecting method according to claim 9, wherein the DNA or RNA probes, which are mutually different in the labels, are hybridized with a plural different specimen DNA or RNA, thereby detecting plural kinds of specimen DNA or RNA.

12. A gene detecting method according to claim 9, wherein the DNA or RNA probes, which have different base sequences and are labelled with different labels, are hybridized with plural sites of a specimen DNA or RNA, thereby detecting the plural sites of the specimen DNA or RNA.

13. A gene detecting method according to claims 4 or 12, wherein in each of said DNA or RNA probes the base at a predetermined position on the DNA or RNA probe is chemically modified so that decomposition by the enzyme having exonuclease activity does not proceed over the base at that position.

14. A gene detecting method according to any of the claims 4 to 13, wherein the length of said DNA or RNA probe is not less than 8 bases and not more than 100 bases.

15. A gene detecting method according to any of the claims 3 to 14, wherein said enzyme having exonuclease activity is exonuclease III, X-exonuclease or Bal 31.

16. A gene detecting method according to any of the claims 2 to 13, wherein the label is at least one of a fluorescence label, a dye or a radioactive label, or a mixture thereof.
